# EUROPEAN PATENT SPECIFICATION

(11) **EP 0 775 495 B1**
(45) Date of publication and mention of the grant of the patent: **07.03.2001**
(21) Application number: 95118582.6
(22) Date of filing: 25.11.1995
(51) Int. Cl.: A61K 39/35, A61K 47/26

(54) **Use of a disaccharide as stabilizer for liquid protein mixtures and liquid protein mixtures containing a disaccharide**
Verwendung eines Disaccharids als Stabilisator für flüssige Proteinmischungen sowie ein Disaccharid enthaltende flüssige Proteinmischungen
Utilisation d'un disaccharide comme stabilisant de mélanges liquides de protéines et mélanges liquides de protéines contenant un disaccharide

(43) Date of publication of application: 28.05.1997
(73) Proprietor: Study Center of Allergy Projects (S.C.A.P.), 2011 KS Haarlem (NL)
(72) Inventor: Stevens, Erik A.M., B-3202 Lubberk-Linden (BE); Cadot, Pascal, B-3360 Bierbeek (BE)
(74) Representative: Fleischer, Holm Herbert, Dr.

(56) References cited:
- EP-A- 0 271 079
- FR-A- 2 068 465
- US-A- 3 577 526
- SOUGH RICHARD: 'Remington: The Science and Practice of Pharmacy, Chapter 82, page 1438', 1995, MACK PUBLISHING, EASTON , PENN

## Description

The present invention relates to the use of a disaccharide such as sucrose as a stabilizer for liquid protein mixtures comprising an agent for preventing microbial growth and a composition containing a liquid protein mixture, a disaccharide preferably sucrose and an agent for preventing microbial growth.

It has been recognized by the medical world for a long time that the physiologically active components in liquid active compositions, in particular in active compositions of biological origin, for example allergenic compositions, tend to chemically degrade. The poor storing stability of such composition makes it difficult, if not impossible, to be able to store them without appropiate stabilizers.

The poor storing stability, in particular as far allergenic extracts from biologically allergenic sources, such as pollens, is concerned is disadvantageously noticeable in practical application due to the danger of wrong dosage. Moreover, allergenic extracts are mixtures of labile and stable proteins. Degradation, therefore, changes not only the overall potency of these extracts but also their composition. This may affect their quality for diagnosis and treatment of allergy. Liquid pharmaceutical active compositions from biologically allergenic sources play an important part as preparations for parenteral administration in the diagnosis and therapy of allergy. Due to the ever increasing symptoms in the field, intensive research into satisfactorily solving the problem of instability in allergenic compositions has been carried out, in particular for such which are optionally parenteraly applied after having been specially treated.

It has been suggested to make allergenic extracts in phosphate or bicarbonate buffer salines and to use glycerol, human serum albumin (HSA) or a detergent like Tween 80® as stabilizing additive.

An effective method of improving the storage stability of liquid compositions of physiologically, active substances was described in US-A-4,600,583 and US-A-4,605,557 corresponding to EP-A-106 727. Therein the stabilizing effect is obtained by adding aliphatic or alicyclic amino substituted carboxylic acids, their pharmaceutically acceptable derivates or salts, respectively, whereby the carboxylic acid unit of the stabilizing compound exhibits a terminal optionally an alkyl substituted amino group. By adding compounds such as tranexamic acid, lysine or E-aminocaproic acid in small amounts, depending on the storage temperature, it was possible to increase the storage stability by two to three times, compared to allergenic extract compositions without the addition of such stabilizers. In EP-A-339 531 the synergetic effect of an additive of formula 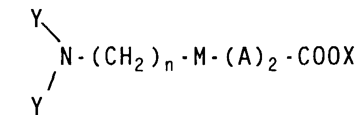 whereby X and Y are independently hydrogen or C₁-C₅-alkyl groups, M is an alkylene or cycloalkylene group, A is a substituted or unsubstituted methylene group, and n is equal to 0 or 1, and the extraction pH value of 5.5 to 6.5 on a liquid allergenic composition is disclosed. The preferred additive is E-aminocaproic acid.

Lee JC. et al discloses in "The stabilization of proteins by sucrose", J. Biol. Chem. 1981:256:7193-201 the stabilizing effect of sucrose in protein solutions under specific conditions.

Due to the consecutive administration of allergenic compositions in the diagnosis and therapy of allergy the solutions containing the allergens are stored in multipuncture vials. Such vials are closed with a rubber cap. For an injection the needle of the syringe is pushed through the rubber cap to take out the desired volume of solution. The rest of solution is kept for subsequent injections. With such a system, contamination of the solution by microorganisms may occur because of the multiple punctures through the cap. Therefore, an agent for preventing microbial growth is added to the solutions.

Commonly, phenol is used as agent for preventing microbial growth in bulk solutions and in final products. (Sough R. JN The Science and Practice of Pharmacy, e.d.A.R. Gennaro. Easton, Penn.: Mack Publishing, 1995, Chapter 82, page 1438). The phenol concentration can range from 0.01 to 0.1, preferably from 0.04 to 0.07 mol/l.

Furthermore, it is known that the above discussed phenomenon of degradation of protein mixtures is accentuated by agents for preventing microbial growth, especially phenol. Thus, there is still a need to minimize the degradation of protein mixtures in liquid form, especially in the presence of antimicrobial agents, such as phenol. Among the above discussed stabilizers for protein mixtures only glycerol was tested in the presence of phenol as disclosed in Ayuso R, Rubio M, Herrera T, Gurbindo C, Carreira J. in Stability of Lolium perenne extract Ann Allergy 1984:53:426-30. But these results were not satisfactory.

US-A-3 577 526 discloses the use of sucrose to stabilise a virulent smallpox vaccine.

Thus, it is the object of the present invention to minimize protein degradation in liquid protein mixtures comprising an agent of preventing microbial growth, such as phenol.

This object has been attained by the use of a disaccharide as stabilizer for liquid protein mixtures comprising an agent for preventing microbial growth and a composition comprising a protein mixture, an agent for preventing microbial growth, a disaccharide and a solvent.

The preferred disaccharide is sucrose.

The composition of the present invention is in liquid form and comprises a suitable non-toxic vehicle, e.g. water.

The protein compounds which may be employed in the practice of the present invention include such substances as allergenic substances, allergenic extracts, sera, toxins, antitoxins. The protein content in the liquid mixture according to the present invention is generally between 0.01 and 10,000 µg/l, preferably 0.1 to 2,000 µg/l.

The preferred protein mixture is an allergenic extract. Whereby the allergenic extract may be selected from extracts of pollen, mites, moulds, animal epithelia, food and latex.

The final composition of this invention may be prepared in any manner considered suitable by the skilled worker practicing the invention. The individual components of the final composition of this invention may be admixed from the desired composition and in some instances where the protein mixture is in a dry state, for example, it may first be reconstituted by the addition of the desired liquid vehicle, for example water. An antimicrobial agent, preferably phenol, will be incorporated for preventing microbial growth in an amount of from 0.01 to 0.1 mol/l, based on the liquid composition. The required amounts of disaccharide, preferably sucrose will also be added, for example by admixing to yield the final storage stable composition of the present invention. Preferably the disaccharide such as sucrose is present in an amount of 0.1 to 1.5 mol/l, most preferably of 0.25 to 1 mol/l.

In a preferred method allergenic materials, for example ryegrass pollen, are extracted using an extraction medium containing water, sucrose and the antimicrobial agent, preferably phenol in the above defined amounts. The extracts are centrifuged to remove residual pollen particles, and then sterilized preferably through a 0.45 µm membrane.

In an alternative method for preparation of a storage stable liquid protein mixture the allergenic material is extracted with water and then centrifuged. Thereafter, the antimicrobial agent, preferably phenol, and sucrose are added in the above defined appropriate amounts. Finally, the liquid composition is sterilized.

By using preparation methods as defined above, storage stable compositions according to the present invention are obtained. Thereby storage stability in the practice of the present invention is defined in that the protein content of a composition containing water as solvent, 0.5% per weight (0.052 mol/l) phenol and sucrose in the above defined appropriate amount after storage for 8 days at 37°C is twice than the protein content of the like composition without sucrose under identical storage conditions.

In a further aspect of the present invention it was found that the protein content of the liquid composition after extraction is considerably increased when a sucrose solution is used as extraction medium in comparison to distilled water as extraction medium, espacially when an anti-microbial agent such as phenol is present. But the protein content is also increased in comparison to distilled water as extraction medium when no phenol is present.

The invention will be further illustrated by the following examples.

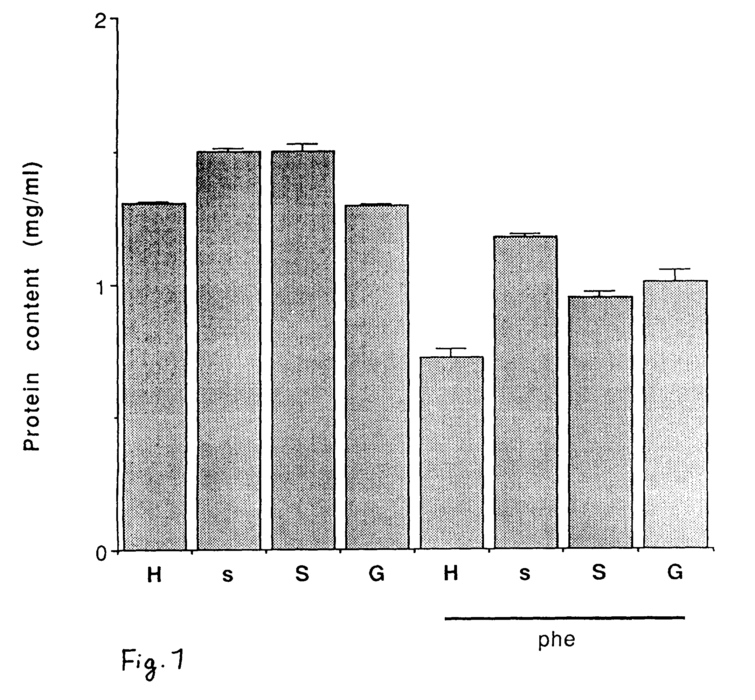

Fig. 1. shows protein content by Coomassie brilliant blue method of ryegrass pollen extracts prepared in distilled water (H), 0.5 M sucrose (s), 1 M sucrose (S), and 1 M glycerol (G), with 0.5% (phe) or without phenol in the extraction medium. Mean ± SD of three experiments.

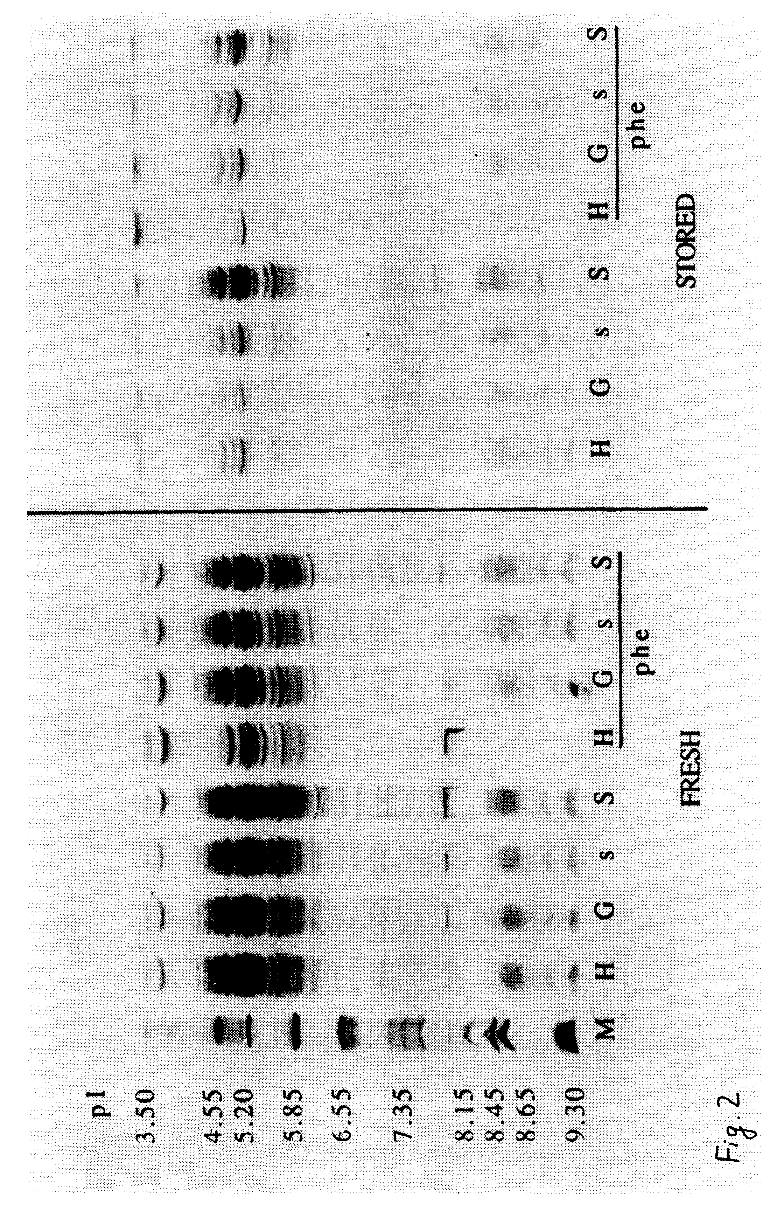

Fig. 2. shows Coomassie brilliant blue-stained IEF gel of ryegrass pollen extracts prepared in distilled water (H), 1 M glycerol (G), 0.5 M sucrose (s), and 1 M sucrose (S), with 0.5% (phe) or without phenol in the extraction medium. The extracts are shown before (fresh) and after storage for 8 days at 37°C (stored). Fresh extracts: 15 µg protein /lane; stored extracts: 5 µg/lane. Markers are in lane M.

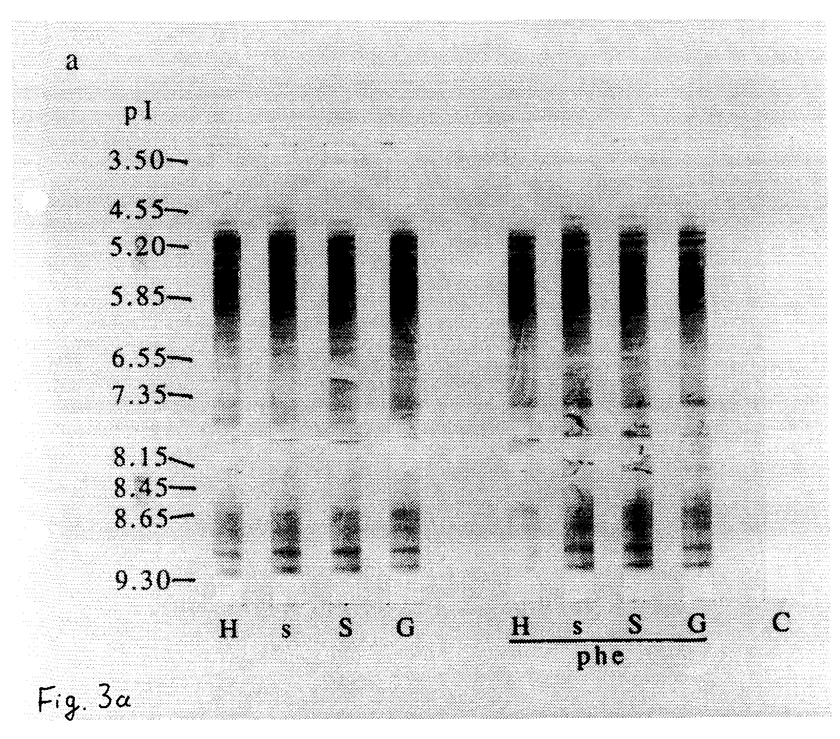

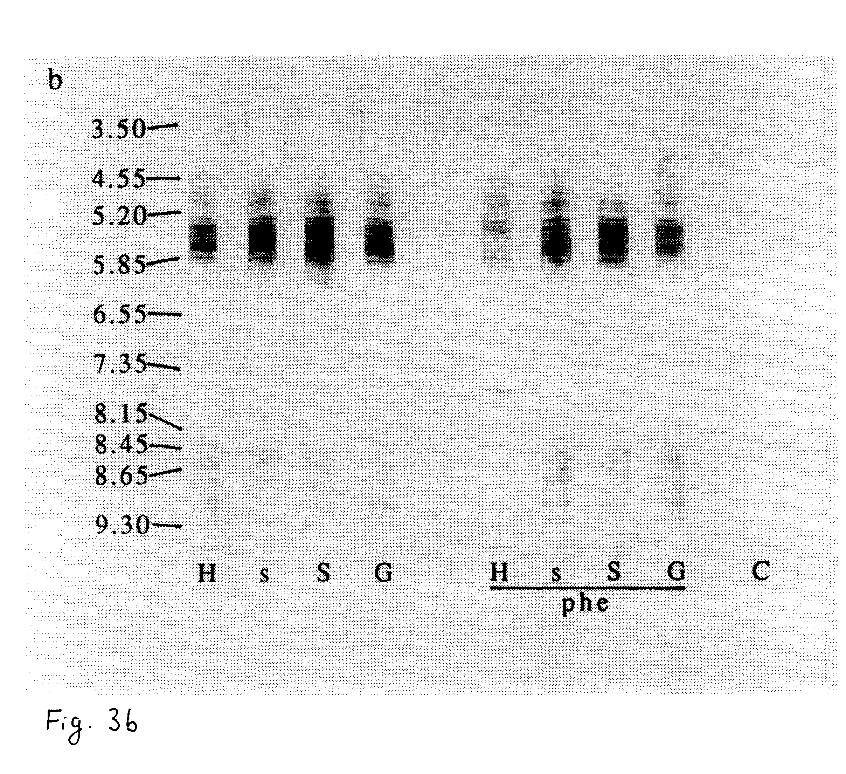

Fig. 3. shows IgE immunoblots from IEF gels. Ryegrass pollen extracts prepared in water (H), 0.5 M sucrose (s), 1 M sucrose (S), and 1 M glycerol (G), with 0.5% phe) or without phenol in the extraction medium are shown before (a) and after storage for 8 days at 37°C (b). Fresh extracts: 5 µg protein/lane; stored extracts: 1 µg/lane. Control strips are in lane C.

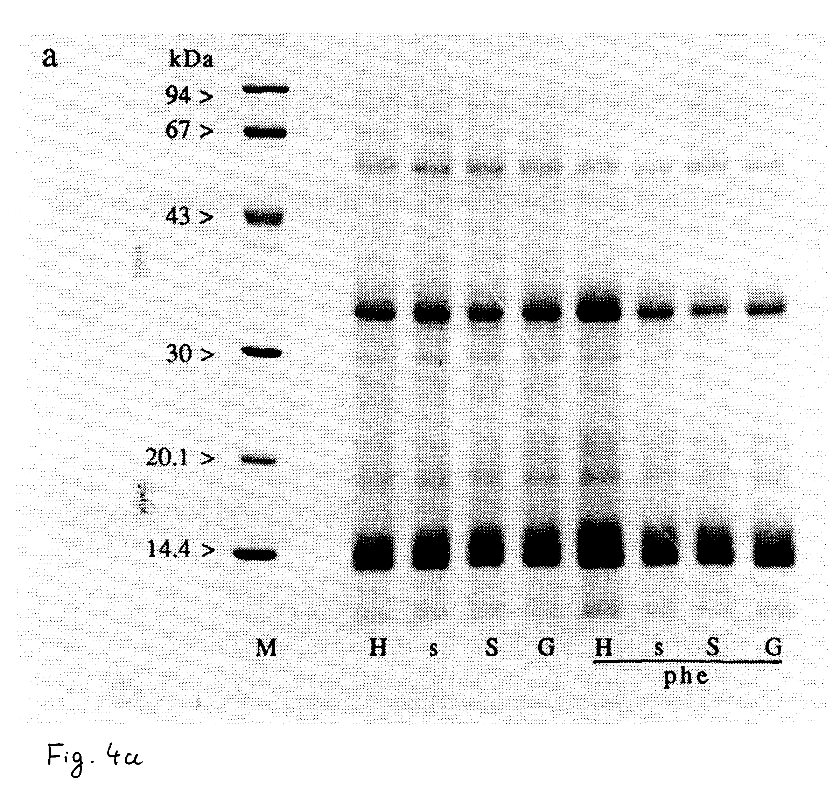

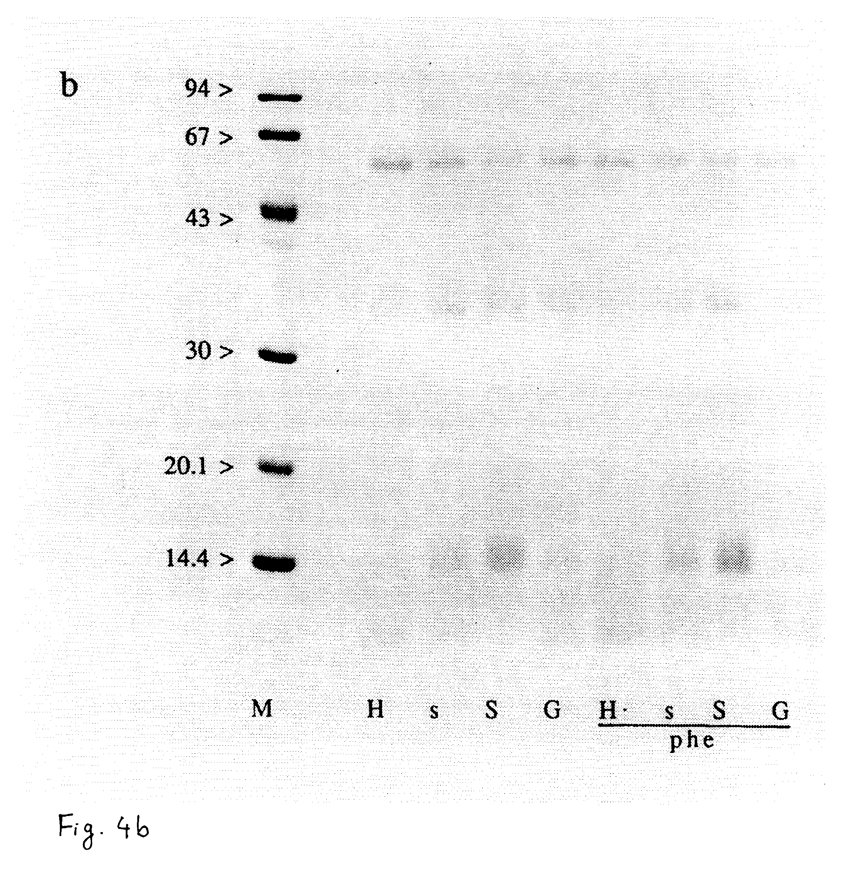

Fig. 4. shows Coomassie brilliant blue-stained SDS-PAGE gel of ryegrass pollen extracts prepared in distilled water (H), 0.5 M sucrose (s), 1 M sucrose (S), and 1 M glycerol (G), with 0.5% (phe) or without phenol in the extraction medium. The extracts are shown before (a) and after sorage for 8 days at 37°C (b). Fresh extracts: 15 µg protein/lane; stored extracts: 5 µg/lane. Molecular weight markers are in lane M.

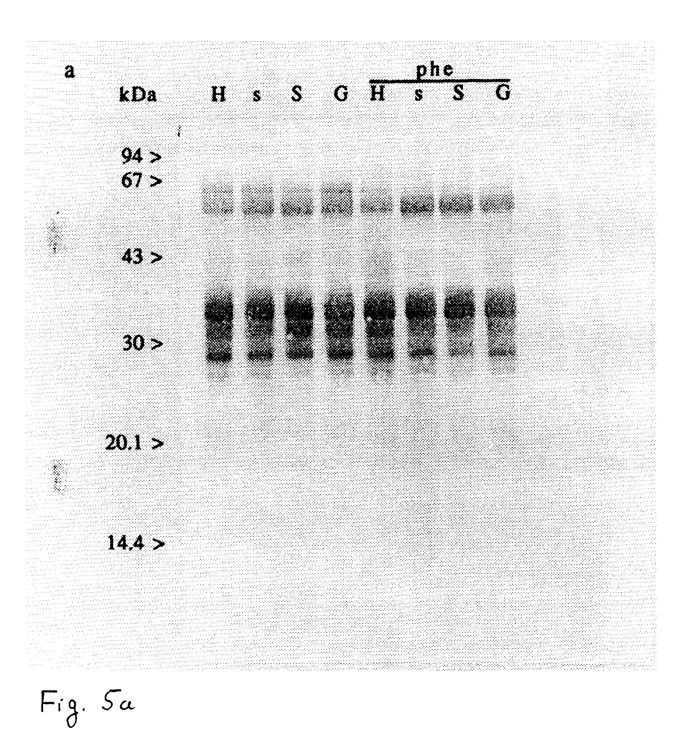

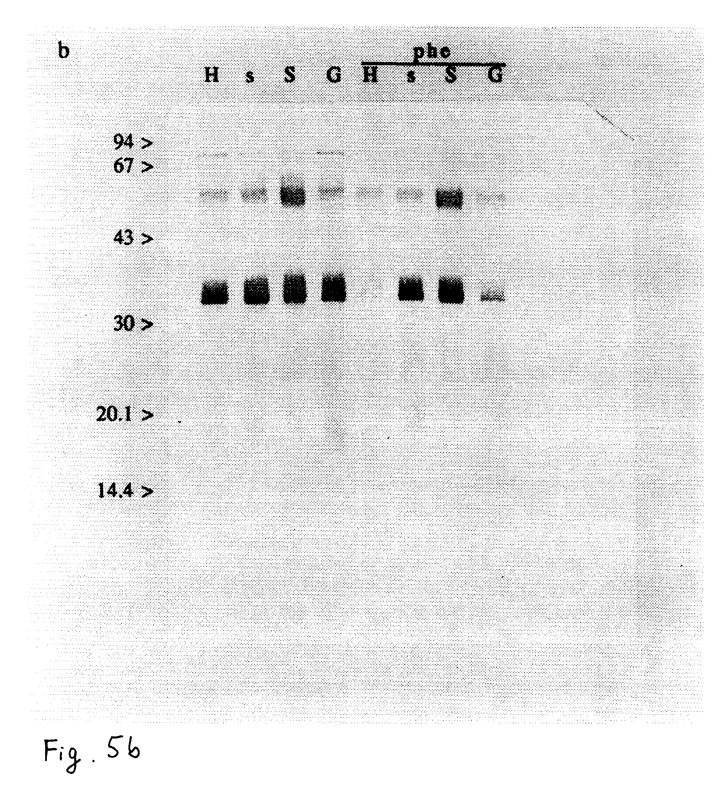

Fig. 5. shows IgE immunoblots from SDS-PAGE gels. Ryegrass pollen extracts prepared in water (H), 0.5 M sucrose (s), 1 M sucrose (S), and 1 M glycerol (G), with 0.5% (phe) or without phenol in the extraction medium are shown before (a) and after storage for 8 days at 37°C (b). Fresh extracts: 5 µg protein/lane; stored extracts: 1 µg/lane.

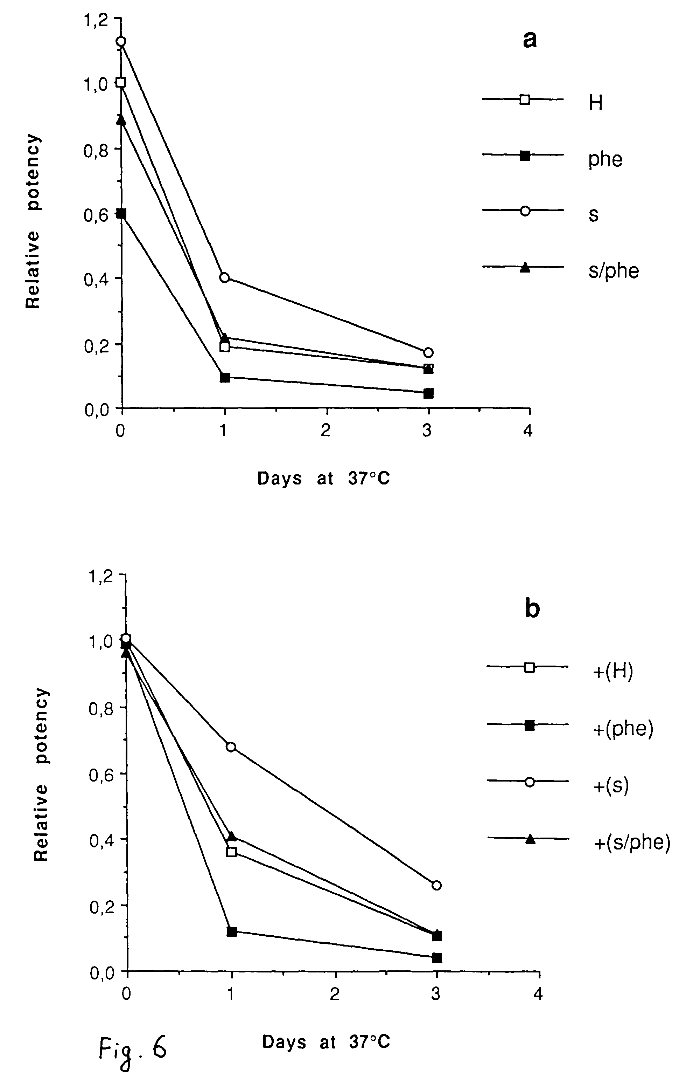

Fig. 6. Results of RAST-inhibition experiments. Graph (a) shows the potency of ryegrass pollen extracts prepared in distilled water (H), 0.5% phenol (phe), 0.5 M sucrose (s), and 0.5% phenol in 0.5 M sucrose (s/phe). Graph (b) shows the potency of ryegrass pollen extracts prepared in water and supplemented after extraction with 0.5% phenol (+(H/phe)), 0.5 M sucrose (+(s)), 0.5% phenol and 0.5 M sucrose (+(s/phe)), or water (+(H)). In both cases the potencies were measured immediately after extraction, and after storage for 1 and 3 days. The fresh extract prepared in water was used as reference.

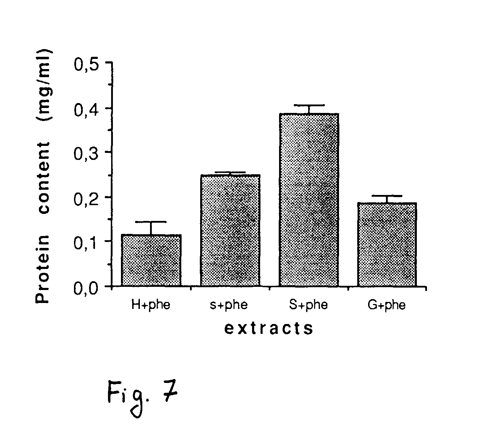

Fig. 7. shows protein content after storage for 8 days at 37°C of ryegrass pollen extracts prepared in distilled water with 0.5% phenol (H+phe), 0.5 M sucrose with 0.5% phenol (s+phe), 1 M sucrose with 0.5% phenol (S+phe), and 1 M glycerol with 0.5% phenol (G+phe), measured by Coomassie blue method. Mean ± SD of three experiments.

### Materials and methods:

### Raw materials:

L. perenne pollen was provided by Haarlems Allergenen Laboratorium (H.A.L.), (Haarlem, The Netherlands, Pharmacia Allergon (Sweden) batch No. 021485101). It was 92% pure and contained <0.5% detectable spores and <0.2% foreign pollen.

### Patient sera:

A total of 106 Lolium perenne-sensitive sera were collected from patients who were clinically reactive to grass pollen. These sera were pooled for immunoblotting experiments. Sera with specific IgE to grass pollen <17.5 Ua/ml (CAP System of Pharmacia, Sweden), or total serum IgE of >2.000 I.U. (CAP system) were rejected. A control serum pool consisted of 3 sera from patients with a monoallergy to house-dust mite (Dermatophagoides pteronyssinus).

### Protein content:

The protein content of extracts was determined by the Coomassie brilliant blue assay (Bio-Rad, California, USA), according to the manufacturer's instructions. Bovine Serum Albumin (BSA) was used as a standard.

### Electrophoresis:

Isoelectric focusing (IEF) was performed using an LKB 2117 Multiphor II™ (LKB-Pharmacia, Sweden), in 5% thin acrylamide gels containing 7.5% ampholytes with pH range 3-10 (Isolab, Ohio, USA) poured on Gel bond® PAG film (FMC, ME, USA). The maximum running conditions were 1500 V, 25 mA, 15 W for a total of 1250 Vh.

Sodium dodecylsulfate polyacrylamide gel electrophoresis (SDS-PAGE) experiments were carried out in 14% acrylamide gels with 5% acrylamide stacking gel, using the discontinuous buffer system described by Laemmli in ("Cleavage of structural proteins during the assembly of the head of bacteriophage T4", Nature 1970:227:680-5). Before running, samples were heated for 5 minutes at 100°C in sample buffer (Tris 125 mmol/l, pH 6.8, glycerol 17.5%, SDS 4%, β-mercaptoethanol 1%, and bromophenol blue 0.002%). A tension of 150 V in the stacking gel and next 350 V in the separation gel was applied until the bromophenol blue reached the lower edge of the gel.

IEF and SDS-PAGE gels were either stained with Coomassie brilliant blue or used for immunoblotting.

### Immunoblotting:

Proteins were electrophoretically transferred from SDS-PAGE gels to polyvinyldifluoride (PVDF) membranes (Immobilon®, Millipore, Massachusetts, USA) in a transfer buffer (25 mmol/l Tris, 192 mmol/l glycine, 20% methanol) at 100 V for 3 h. For IEF gels, proteins were transferred to PVDF membranes by press-blotting. In both cases the membranes were next immersed for 2 h in blocking solution, consisting in 0.2% nonfat dry milk in phosphate buffered saline (PBS-NFDM 0.2%). Membrane strips were cut and incubated overnight in sera diluted 1/8 in PBS-NFDM 0.1%. Bound IgE was detected by monoclonal mouse antihuman IgE antibodies (CLB, The Netherlands) diluted 1/1000 in PBS-NFDM 0.1% (1 h, room temperature), followed by peroxidase-conjugated rat antimouse light-chain kappa antibodies (CLB, The Netherlands), diluted 1/2000 in PBS-NFDM 0.1% (2 h, room temperature). Bands were revealed by incubation in the peroxidase substrate 3,3',5,5'-tetramethylbenzidine (TMB, KPL, Maryland, USA).

### RAST-inhibition tests:

RAST discs were prepared by coupling an aqueous ryegrass pollen extract to CNBr-activated Whatman cellulose paper discs according to the method of Ceska et al in "Radioimmunosorbent assay of allergens", J Allergy Clin Immunol 1972:49:1-9. For RAST-inhibition, 50 µl of allergenic extract was incubated with 50 µl of serum pool. After 2 h, RAST discs were added to the tubes. After 3 h of incubation, the discs were washed 3 times and then incubated overnight with 50 µl of radio-iodinated anti-IgE antibodies (Pharmacia, Sweden). Every dilution of every sample was tested in triplicate. Relative potencies were calculated according to Anderson and Baer in "RAST-inhibition procedure", Bethesda, Maryland, Technical bulletin, Bureau of Biologics, Food and Drug Administration, 1981.

### Example 1

### Effect of sucrose during extraction:

To assess the protective effect of sucrose on extraction, L. perenne pollen was extracted 1 g/10 ml in water (H) in 1 M glycerol (G) in 0.5 M (s) or 1 M (S) sucrose with or without the presence of 0.5 wt-% phenol for 3 h at room temperature under constant agitation. These extracts were next centrifuged (17.500 g, 2x30 min, 4°C) to remove pollen particles. Afterwards they were sterilized through a 0.45 µm membrane, aliquoted and kept at -20°C until use.

Fig. 1 shows that the total protein content of phenolfree sucrose-containing extracts was increased equally by about 13% for 0.5 and 1 M sucrose. Phenol was detrimental for the proteins in all preparations. However, in the presence of either sucrose (0.5 M or 1 M) or 1 M glycerol the extraction yield was higher. The highest total protein content for phenolated extracts was obtained with 0.5 M sucrose.

A Coomassie brilliant blue-stained IEF gel (Fig. 2, left part) demonstrated that phenol-free extracts made with or without sucrose or with glycerol displayed a similar pattern of 24 bands, although some bands appeared to be somewhat more intense for extracts containing 1 M sucrose. In phenolated extracts without any stabilizer, all bands in the basic pI range disappeared and a general fading of the pattern was observed, whereas in the presence of either sucrose or glycerol the patterns were similar to those of phenol-free extracts.

The corresponding immunoblots (Fig. 3a) showed similar patterns with 7 bands in the pI range 4.50-5.90 and 4 basic bands for all extracts, except for the simple phenolated extract, which showed weak binding in the basic part of the pattern.

SDS-PAGE gel analysis demonstrated a same pattern of 9 bands for all phenol-free extracts (Fig. 4a). similar patterns were obtained for phenolated extracts except for fading of bands at 83 and 66 KDa. A slightly increased intensity of bands at 34 (Lol p1) and 14 kDa was noticed for the simple phenolated extract.

On corresponding immunoblots (Fig. 5a) high IgE-binding was found in all extracts for proteins at 83, 57, 34, 32, 29, and 27 kDa, among which Lol p 1 (at 34 kDa) and Lol p 4 (at 57 kDa) are well-known allergens. The band at 66 kDa had faded for all phenolated extracts.

A 0.5 M sucrose extract and a simple aqueous extract, with or without 0.5% phenol, were compared by RAST-inhibition (Fig. 6a). Immediately after extraction the 0.5 M sucrose extract was somewhat more potent than the phenolfree aqueous extract, but the difference in potency expressed as percentage was minimal. The potency of extracts prepared in phenolated media was lower than for their phenol-free counterparts. Importantly, the potency of a 0.5 M sucrose- and phenol-containing extract was equivalent to that of a phenol-free aqueous extract.

### Example 2

### Effect of sucrose during storage:

The extracts as described in Example 1 were subjected to forced degradation upon storage in sterile vials at 37°C for 8 days.

In Coomassie brilliant blue-stained IEF gels (Fig. 2, right part), bands tended to disappear for all stored extracts. The simple phenol-containing extract was most degraded, whereas bands remained more intensely stained in a 1 M sucrose phenol-free extract. In phenolated extracts, the presence of either glycerol or sucrose had a significant preserving effect, particularly for the phenol-sensitive basic proteins. Sucrose 1 M was the best stabilizer in extracts prepared with or without phenol, while 0.5 M sucrose and 1 M glycerol were about equivalent. Only one band at pI 3 was more prominent for the extract containing only phenol than for the stabilized extracts.

On IEF immunoblots (Fig. 3b), patterns did not differ appreciably among aqueous, glycerol-, or sucrose-containing phenol-free extracts, and stabilized phenolated extracts. The simple phenolated extract showed a less intense reaction in the pI region 4.50-5.90. Furthermore, the band at pI 3 and the basic bands did not bind IgE.

The Coomassie brilliant blue-stained SDS-PAGE patterns of stored extracts were clearly different after storage (Fig. 4b). Lol p 4 (at 57 kDa) seemed to be relatively resistant to degradation, even in the presence of phenol, but the majority of the other bands had faded out. Phenolated or not, extracts containing sucrose (0.5 M or 1 M) were better preserved. Particularly, the major allergen Lol p 1 at 34 kDa, which was barely visible in a simple aqueous extract after 8 days of storage, was partially preserved by sucrose and glycerol. In addition, Lol p 1 appeared to be sensitive to phenol. In phenolated extracts, it was partly protected by sucrose, particularly at 1 M concentration, but not by 1 M glycerol.

IgE immunoblot patterns of stored phenol-free extracts (Fig. 5b) differed for two bands at 66 and 57 kDa (Lol p 4), which were more prominent in the extract containing 1 M sucrose. For phenol-free extracts without sucrose, Lol p 1, that was not stained by Coomassie blue, appeared to still be present in sufficient amounts to be detected by specific IgE. This was however not the case for phenolated extracts, for which the loss of Lol p1 was confirmed, in the absence of any stabilizer. Among the stabilizers, sucrose (0.5 M and 1 M) was more efficient at preserving Lol p 1 than 1 M glycerol.

Potency measurements by RAST-inhibition (Fig. 6a) demonstrated that a phenolated extract in 0.5 M sucrose was almost equivalent to a simple non-phenolated extract, after 1 and 3 days at 37°C. It degraded faster, however, than a sucrose-containing phenol-free extract.

Measurement of protein content after storage for 8 days at 37°C of phenolated ryegrass pollen extracts (Fig. 7) showed that the protein content of sucrose-containing extracts was at least twice in comparison to the protein content of a like composition without sucrose, indicating the improved storage stability of sucrose-containing compositions in comparison to glycerol-containing compositions.

### Example 3

Storage stability of extracts supplemented with sucrose and/or phenol after extraction.

Allergenic extracts of L. perenne pollen were prepared (1 g/10 ml) by extracting in distilled water for 3 hours at room temperature and constant agitation but after centrifugation the extracts were then divided into four equal parts, to one of them sucrose was added to a final concentration of 0.5 M (+s extract), the second part was supplemented with 0.5% phenol (+phe extract), and the third with 0.5 M sucrose and 0.5 wt-% phenol (+s/phe extract). The fourth part contained no additive (+H extract) but was adjusted with distilled water to the same volume as the other parts to compensate for the increase in volume mainly due to the addition of sucrose. These extracts were sterilized and stored as described in Example 1 and/or Example 2. The evolution of potency during storage, shown in Fig 6b, was only evaluated for those extracts. After 1 and 3 days at 37°C the potency of the (+s extract) was about twofold that of the (+H) extract. After 1 day the (+phe) extract lost about 90% of its initial potency, while its counterpart with sucrose lost only 60%, i.e. the same loss as a (+H) extract. After 3 days, the potency of the (+s/phe) extract was still twofold that of the (+phe) extract, though they both were very low. After 8 days at 37°C the potency was too low for all extracts to be evaluated.

The above cited examples confirm the detrimental effect of phenol during extraction on proteins. Not only is the protein content of a phenolated extract decreased, but all the bands, particularly the basic proteins, are less intense or even absent in an IEF gel. However, these basic proteins still remain slightly visible on immunoblots, due to the higher sensitivity of the method. When sucrose is present in the extraction medium the proteins are partly protected from phenol. Indeed, protein content increases and bands on IEF gels and corresponding IgE immunoblots are more intense and comparable to those of phenol-free extracts. Although, in SDS-PAGE gel analysis, there is no marked difference between phenol-containing extracts with and without sucrose, the stabilizing effect over phenol is clearly demonstrated by RAST-inhibition potency measurements. A phenolated extract with sucrose equals a non-phenolated aqueous extract, thus confirming the impression gained from the comparison of IEF immunoblots that fragile proteins are denatured by phenol during the extraction process.

Sucrose also stabilizes phenolated allergenic extracts during storage. Particularly, the basic proteins, which are very sensitive to phenol, are still preserved by sucrose after 8 days at 37°C. The peculiar sensitivity of Lol p 1 which is the dominant allergenic protein in ryegrass pollen and therefore of great importance for the diagnosis and hypo- and desensibilization therapy of allergies for phenol is apparent from SDS-PAGE gels and immunoblots of stored extracts, as is the usefulness of sucrose to counteract its degradation.

To evaluate the practical usefulness of sucrose its activity was compared with that of other stabilizers. Because of its widespread use, glycerol was selected for that purpose. The stabilizing properties of sucrose and glycerol during extraction and subsequent storage, in the presence or absence of 0.5 wt-% phenol, were compared. The results showed that sucrose is a better stabilizer than glycerol at equimolar concentrations. We found that 1 M glycerol is similar to 0.5 M sucrose, considering the extract patterns in electrophoresis experiments. However, 1 M glycerol is much less potent than 0.5 M sucrose at preserving Lol p 1 from degradation by phenol.

The results of a RAST-inhibition experiment demonstrate that the potency of a phenol-containing extract in 0.5 M sucrose is roughly equivalent to that of an aqueous extract without phenol even after 3 days at 37°C. the same applies for an extract supplemented with sucrose and phenol after extraction. The dramatic loss in potency of an extract supplemented with 0.5% phenol reflects the fast denaturation of the most fragile allergens. This denaturation is slower in the presence of sucrose.

Thus, the results of the gel electrophoresis, immunoblotting and RAST-inhibition experiments all demonstrate that sucrose counteracts to a significant extent the toxic effect of phenol on allergenic extracts. Additionally, sucrose is more potent as stabilizer in comparison to glycerol.

## Claims

1. Use of a disaccharide as stabilizer for liquid protein mixtures comprising an agent for preventing microbial growth.

2. Use of claim 1
**characterized in that**
the protein mixture is an aqueous mixture.

3. Use of any of claims 1 or 2
**characterized in that**
the protein content in the liquid mixture is 0.01 to 10,000 µg/l.

4. Use of any of the preceding claims
**characterized in that**
the protein mixture is an allergenic extract.

5. Use of claim 4
**characterized in that**
the allergenic extract is selected from extracts of pollen, mites, animal epithelia, food, and latex.

6. Use of any of the preceding claims
**characterized in that**
the concentration of the agent for preventing microbial growth in the liquid protein mixture is from 0.01 to 0.1 mol/l.

7. Use of any of the preceding claims
**characterized in that**
the agent for preventing microbial growth is phenol.

8. Use of any of the preceding claims
**characterized in that**
the disaccharide is present in an amount of 0.1 to 1.5 mol/l.

9. Use of claim 8
**characterized in that**
the disaccharide is sucrose.

10. A composition comprising:
- a protein mixture,
- an agent for preventing microbial growth,
- a disaccharide, and
- a solvent.

11. The composition of claim 10
**characterized in that**
the solvent comprises water.

12. The composition of any of claims 10 or 11
**characterized in that**
the protein mixture is present in an amount of 0.01 to 10,000 µg/l.

13. The composition of any of claims 10 - 12
**characterized in that**
the protein mixture is an allergenic extract.

14. The composition of claim 13
**characterized in that**
the allergenic extract is selected from extracts of pollen, mites, moulds, animal epithelia, food and latex.

15. The composition of any of claims 10 - 14
**characterized in that**
the concentration of the agent for preventing microbial growth in the liquid protein mixture is from 0.01 to 0.1 mol/l.

16. The composition of any of claims 10 - 15
**characterized in that**
the agent for preventing microbial growth is phenol.

17. The composition of any of claims 10 - 16
**characterized in that**
the disaccharide is present in an amount of 0.1 to 1.5 mol/l.

18. The composition of claim 17
**characterized in that**
the disaccharide is sucrose.

## Patentansprüche

1. Verwendung eines Disaccharids als Stabilisator für flüssige Proteinmischungen, enthaltend ein Mittel zur Verhinderung des Wachstums von Mikroben.

2. Verwendung gemäß Anspruch 1, dadurch gekennzeichnet, daß die Proteinmischung eine wäßrige Mischung ist.

3. Verwendung gemäß einem der Ansprüche 1 oder 2, dadurch gekennzeichnet, daß der Proteingehalt in der flüssigen Mischung 0,01 bis 10.000 µg/l ist.

4. Verwendung gemäß einem der vorstehenden Ansprüche, dadurch gekennzeichnet, daß die Proteinmischung ein allergener Extrakt ist.

5. Verwendung gemäß Anspruch 4, dadurch gekennzeichnet, daß der allergene Extrakt ausgewählt ist aus Extrakten von Pollen, Milben, tierischen Ephitelien, Nahrungsmitteln und Latex.

6. Verwendung gemäß einem der vorangehenden Ansprüche, dadurch gekennzeichnet, daß die Konzentration des Mittels zur Verhinderung von Wachstum von Mikroben in der flüssigen Proteinmischung von 0,01 bis 0,1 mol/l ist.

7. Verwendung gemäß einem der vorangehenden Ansprüche, dadurch gekennzeichnet, daß das Mittel zur Verhinderung des Wachstums von Mikroben Phenol ist.

8. Verwendung gemäß einem der vorangehenden Ansprüche, dadurch gekennzeichnet, daß das Disaccharid in einer Menge von 0,1 bis 1,5 mol/l vorliegt.

9. Verwendung gemäß Anspruch 8, dadurch gekennzeichnet, daß das Disaccharid Sucrose ist.

10. Eine Zusammensetzung, enthaltend:
- eine Proteinmischung,
- ein Mittel zur Verhinderung von Wachstum von Mikroben,
- ein Disaccharid, und
- ein Lösemittel.

11. Lösemittel gemäß Anspruch 10, dadurch gekennzeichnet, daß das Lösemittel Wasser enthält.

12. Zusammensetzung gemäß einem der Ansprüche 10 oder 11, dadurch gekennzeichnet, daß die Proteinmischung in einer Menge von 0,01 bis 10.000 pg/1 vorliegt.

13. Zusammensetzung gemäß einem der Ansprüche 10 bis 12, dadurch gekennzeichnet, daß die Proteinmischung ein allergener Extrakt ist.

14. Zusammensetzung gemäß Anspruch 13, dadurch gekennzeichnet, daß der allergene Extrakt ausgewählt ist aus Extrakten von Pollen, Milben, Schimmelpilzen, tierischen Epithelien, Nahrungsmitteln und Latex.

15. Zusammensetzung gemäß einem der Ansprüche 10 bis 14, dadurch gekennzeichnet, daß die Konzentration des Mittels zur Verhinderung des Wachstums von Mikroben in dem flüssigen Proteingemisch von 0,01 bis 0,1 mol/1 ist.

16. Zusammensetzung gemäß einem der Ansprüche 10 bis 15, dadurch gekennzeichnet, daß das Mittel zur Verhinderung des Wachstums von Mikroben Phenol ist.

17. Zusammensetzung gemäß einem der Ansprüche 10 bis 16, dadurch gekennzeichnet, daß das Disaccharid in einer Menge von 0,1 bis 1,5 mol/l vorliegt.

18. Zusammensetzung gemäß Anspruch 17, dadurch gekennzeichnet, daß das Disaccharid Sucrose ist.

## Revendications

1. Utilisation d'un disaccharide comme stabilisant pour des mélanges liquides de protéines comprenant un agent pour empêcher la croissance microbienne.

2. Utilisation selon la revendication 1, caractérisée en ce que le mélange de protéines est un mélange aqueux.

3. Utilisation selon les revendications 1 ou 2, caractérisée en ce que la teneur en protéines dans le mélange liquide est de 0,01 à 10 000 µg/l.

4. Utilisation selon l'une quelconque des revendications précédentes, caractérisée en ce que le mélange de protéines est un extrait allergène.

5. Utilisation selon la revendication 4, caractérisée en ce que l'extrait allergène est choisi parmi des extraits de pollen, d'acariens, d'épithéliums animaux, de nourriture, et de latex.

6. Utilisation selon l'une quelconque des revendications précédentes, caractérisée en ce que la concentration de l'agent pour empêcher la croissance microbienne dans le mélange liquide de protéines est de 0,01 à 0,1 mole/l.

7. Utilisation selon l'une quelconque des revendications précédentes, caractérisée en ce que l'agent pour empêcher la croissance microbienne est le phénol.

8. Utilisation selon l'une quelconque des revendications précédentes, caractérisée en ce que le disaccharide est présent en une quantité de 0,1 à 1,5 mole/l.

9. Utilisation selon la revendication 8, caractérisée en ce que le disaccharide est le saccharose.

10. Composition comprenant :
- un mélange de protéines,
- un agent pour empêcher la croissance microbienne,
- un disaccharide, et
- un solvant.

11. Composition selon la revendication 10, caractérisée en ce que le solvant est l'eau.

12. Composition selon l'une quelconque des revendications 10 ou 11, caractérisée en ce que le mélange de protéines est présent en une quantité de 0,01 à 10 000 ug/1.

13. Composition selon l'une quelconque des revendications 10 à 12, caractérisée en ce que le mélange de protéines est un extrait allergène.

14. Composition selon la revendication 13, caractérisée en ce que l'extrait allergène est choisi parmi des extraits de pollen, d'acariens, d'épithéliums animaux, de nourriture, et de latex.

15. Composition selon l'une quelconque des revendications 10 à 14, caractérisée en ce que la concentration de l'agent pour empêcher la croissance microbienne dans le mélange liquide de protéines est de 0,01 à 0,1 mole/1.

16. Composition selon l'une quelconque des revendications 10 à 15, caractérisée en ce que l'agent pour empêcher la croissance microbienne est le phénol.

17. Composition selon l'une quelconque des revendications 10 à 16, caractérisée en ce que le disaccharide est présent en une quantité de 0,1 à 1,5 mole/l.

18. Composition selon la revendication 17, caractérisée en ce que le disaccharide est le saccharose.
